# FASCICULE DE BREVET EUROPEEN

(11) **EP 1 466 590 B1**
(45) Date de publication et mention de la délivrance du brevet: **26.09.2007**
(21) Numéro de dépôt: 04290756.8
(22) Date de dépôt: 22.03.2004
(51) Int. Cl.: A61Q 19/00, A61K 8/49, A61K 8/60, A61K 31/35, A61K 31/70

(54) **Utilisation cosmétique de nouveaux agents desquamants**
Kosmetische Verwendung von neuen abschuppenden Mitteln
Cosmetic use of new desquamative agents

(30) Priorité: 08.04.2003 FR 0304349
(43) Date de publication de la demande: 13.10.2004
(73) Titulaire: L'ORÉAL, 75008 Paris (FR)
(72) Inventeur: Dalko, Maria, 91190 Gif sur Yvette (FR); Cavezza, Alexandre, 93290 Tremblay-en-France (FR); Bernard, Dominique, 75015 Paris (FR)
(74) Mandataire: Kromer, Christophe

(56) Documents cités:
- DE-B- 1 288 612
- US-A- 2 522 966
- US-A- 4 299 845
- US-A- 5 653 970
- GÜNTER WULFF ET AL: "On the synthesis of C-glycosyl compounds containing double bonds without the use of protecting groups" CARBOHYDRATE RESEARCH., vol. 257, 1994, pages 81-95, XP002263620 ELSEVIER SCIENTIFIC PUBLISHING COMPANY. AMSTERDAM., NL ISSN: 0008-6215

## Description

La présente invention se rapporte à des compositions adaptées à une application topique sur la peau ou le cuir chevelu contenant, dans un milieu physiologiquement acceptable, au moins un composé de formule donnée. Elle concerne également un procédé de traitement cosmétique comprenant l'application topique de ces compositions, ainsi que de nouveaux dérivés C-glycosides.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Au cours du processus dit de kératinisation, les kératinocytes situés dans la couche basale de l'épiderme se multiplient et croissent, poussant ainsi les cellules épidermiques plus anciennes vers le haut et vers la surface de l'épiderme. Lors de ce déplacement, ces cellules s'aplatissent et se différencient pour former de la kératine. Les cellules mortes superficielles résultant de ce processus de kératinisation (cornéocytes) constituent la couche cornée de l'épiderme où elles sont séparées par des couches lipidiques et reliées entre elles par des liaisons protéiniques (cornéosomes). Ces cellules mortes sont progressivement éliminées de la surface de la peau et remplacées par de nouvelles cellules kératinisées.

Dans la peau jeune et saine, la desquamation de la peau qui se produit ainsi est caractérisée par l'élimination de cellules individuelles ou de petits amas cellulaires. Au contraire, avec l'âge ou dans le cas de certaines pathologies, la desquamation peut être altérée, en ce sens qu'un excès de matière kératinique se forme à la surface de la peau, résultant soit en une élimination du stratum corneum sous forme de squames (vieillissement cutané, peau sèche), soit en une obstruction des follicules sébacés (acné).

Le document US-A-4 299 845 décrit l'utilisation des dérivés de malonamide pour le traitement de l'acné.

L'utilisation d'agents desquamants, tels que les α-hydroxyacides et les β-hydroxyacides (en particulier l'acide salicylique), est donc généralement indiquée dans le traitement cosmétique ou dermatologique des désordres cutanés précités.

Il n'en demeure pas moins que le désir de conserver une apparence jeune et/ou une peau saine conduit toujours à la recherche incessante de nouveaux composés et/ou de nouvelles compositions permettant de maintenir ou d'améliorer l'apparence de la peau.

Or, la Demanderesse a découvert, de manière surprenante et inattendue, que certains composés, en particulier des dérivés C-glycosides, présentaient des propriétés desquamantes permettant d'envisager leur utilisation dans la prévention ou le traitement des signes du vieillissement cutané, de la peau sèche, et de l'acné.

La présente invention a donc pour objet une composition adaptée à une application topique sur la peau ou le cuir chevelu contenant, dans un milieu physiologiquement acceptable, au moins un composé répondant à la formule (I) suivante : 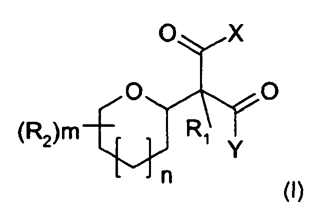 dans laquelle :
R₁ représente un atome d'hydrogène, ou un groupement choisi parmi :
   - un groupe alkyle en C₁-C₁₂, saturé ou insaturé, linéaire, cyclique ou ramifié, éventuellement substitué par un ou plusieurs groupes choisis parmi -OR, -SR, -COOR, - NRR', halogène, sulfate, phosphate, glycoside, aryle et hétérocycle,
      où R et R' représentent, indépendamment l'un de l'autre, un atome d'hydrogène ou un groupe alkyle en C₁-C₁₂, saturé ou insaturé, linéaire, cyclique ou ramifié,
   - un atome d'halogène, par exemple un atome de fluor, chlore, brome ou iode,
   - un groupe aryle éventuellement substitué par un ou plusieurs groupes choisis parmi : -
      OR, -SR, -COOR, -NRR', halogène, sulfate et phosphate où R et R' ont la signification indiquée ci-dessus;
R₂ représente un groupement choisi parmi :
   - R₂₁ où R₂₁ a la définition donnée ci-dessus pour R₁,
   - OR₂₂, où R₂₂ a la définition donnée ci-dessus pour R₁ excepté halogène,
   - OR₂₃ où R₂₃ est un groupe sulfate, phosphate, glycoside, alkylcarbonyle ou un hétérocycle,
   - NR₂₄R₂₅ où R₂₄ et R₂₅ représentent indépendamment un groupe ayant l'une des défintions données ci-dessus pour R₁, excepté halogène,
   - NR₂₆R₂₇ où R₂₆ et R₂₇ représentent indépendamment un radical glycoside ou alkylcarbonyle ou un hétérocycle,
   - un groupe sulfate ou phosphate ;
X et Y représentent indépendamment l'un de l'autre un radical -OR₃ ou -NR₃R₄, où R₃ et R₄ sont indépendamment choisis parmi :
   - un atome d'hydrogène,
   - un groupe alkyle en C₁-C₁₂, saturé ou insaturé, linéaire, cyclique ou ramifié, éventuellement substitué par un ou plusieurs groupes choisis parmi -OR, -SR, -COOR, - NRR', halogène, sulfate, phosphate, glycoside, aryle et hétérocycle, où R et R' ont la signification ci-dessus,
   - un groupe aryle éventuellement substitué par un ou plusieurs groupes choisis parmi : -
      OR, -SR, -COOR, -NRR', halogène, sulfate et phosphate où R et R' ont la signification ci-dessus,
   - ou R₃ et R₄ forment ensemble un cycle comportant de 5 à 7 atomes avec l'atome d'azote auquel ils sont reliés,
   ou X et Y forment un cycle de 6 à 7 atomes de carbone avec les trois atomes de carbone les séparant;
n est un entier égal à 0 ou 1 ; et
m est un entier compris entre 0 et 4.

Selon une forme d'exécution préférée de l'invention, dans la formules (I) ci-dessus, l'une au moins des conditions suivantes est satisfaite :
- R₁ est un atome de fluor ou d'hydrogène ou un radical alkyle non substitué ou benzyle,
- R₂ est un groupe hydroxyle, hydroxyalkyle ou alkyle ou un reste de sucre,
- X et Y sont des groupes -NR₃R₄ où R₃ et R₄ sont indépendamment choisis parmi un atome d'hydrogène et un radical méthyle, éthyle, n-propyle ou isopropyle, et
- n est égal à 1.

Selon une forme d'exécution particulièrement préférée de l'invention, le composé de formule (I) est un dérivé C-glycoside répondant à la formule (II) suivante : 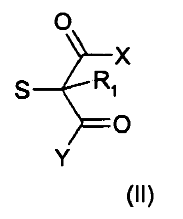 dans laquelle :
- S représente un monosaccharide ou un polysaccharide comportant jusqu'à 20 unités sucre, sous forme pyranose et/ou furanose et de série L et/ou D, ledit mono- ou polysaccharide présentant au moins une fonction hydroxyle libre,
- la liaison S-C représente une liaison de nature C-anomérique,
- R₁, X et Y ont la signification indiquée précédemment.

Avantageusement, les monosaccharides préférés sont choisi parmi le D-glucose, le D-galactose, le D-mannose, le D-xylose, le D-lyxose, le L-fucose, L-arabinose, le L-rhamnose, l'acide D-glucuronique, l'acide D-galacturonique, l'acide D-iduronique, la N-acétyl-D-glucosamine et la N-acétyl- D-galactosamine.

Avantageusement encore, les polysaccharides préférés contiennent jusqu'à 6 unités sucre et sont notamment choisis parmi le D-maltose, le D-lactose, le D-cellobiose, le D-maltotriose, un disaccharide associant un acide uronique choisi parmi l'acide D-iduronique ou l'acide D-glucuronique avec une hexosamine choisi parmi la D-galactosamine, la D-glucosamine, la N-acétyl-D-galactosamine, la N-acétyl-D-glucosamine, un oligosaccharide contenant au moins un xylose avantageusement choisis parmi le xylobiose, le méthyl-(β-xylobioside, le xylotriose, le xylotétraose, le xylopentaose et le xylohexaose et préférentiellement le xylobiose qui est composé de deux molécules de xylose liés par une liaison 1-4.

Selon une forme plus préférentielle encore de l'invention, le composé de formule (I) est un dérivé C-glycoside répondant à la formule (III) : 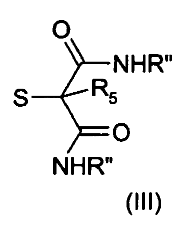 dans laquelle :
- S a la signification indiquée précédemment,
- R₅ désigne :
   un groupe alkyle en C₁-C₁₂, saturé ou insaturé, linéaire, cyclique ou ramifié, non substitué ou
   un radical benzyle, ou
   un atome d'halogène, de préférence un atome de fluor ;
- R" désigne un atome d'hydrogène ou un groupe alkyle en C1-C12, saturé ou insaturé, linéaire, cyclique ou ramifié, éventuellement substitué par un ou plusieurs groupes choisis parmi -OR, -SR, -COOR, -NRR', halogène, sulfate, phosphate, glycoside, aryle et hétérocycle.

La liaison C-anomèrique dans les formules (II) et (III) peut être α ou β.

Selon une forme d'exécution particulièrement préférée de l'invention, les composés de formule (III) sont tels que R₅ est un groupe benzyle ou méthyle et R" est un groupe méthyle.

Les composés de formule (III) étant nouveaux à la connaissance de la Demanderesse, la présente invention a donc également pour objet ces nouveaux composés.

L'invention concerne également les isomères optiques et/ou géométriques des composés de formules (I), (II) et (III), seuls ou en mélange en toutes proportions, ainsi que les sels physiologiquement acceptables de ces composés.

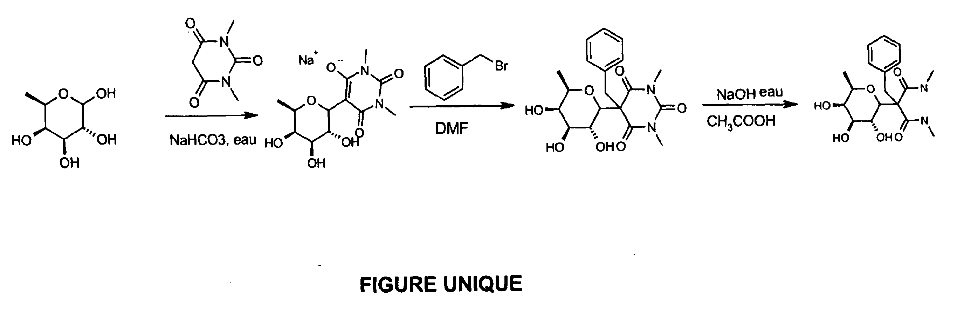

Le Tableau 1 ci-après rassemble des exemples de composés utilisables dans la présente invention. Ces composés peuvent être préparés, par exemple, selon le procédé décrit par GONZALES M.A. et al., Carbohydrate Research, 158 (1986 pp. 53-66 et par WULFF G. et al dans Carbohydrate Research 257 (1994) pp. 81-95, ainsi que dans l'Exemple 1 ci-après par référence à la Figure 1 annexée.

Les composés de la présente application ont déjà été décrites dans les documents DE-A-1 288 612 et US-A-2 522 966. Ils sont utilisés comme intermédiaires pour la synthèse d'autres composés.

**TABLEAU 1**

| **Composé** | **Formule** | **R1** | **R5** | **R2** | **m** | **X, Y** | **R"** | **n** |
|---|---|---|---|---|---|---|---|---|
| A | 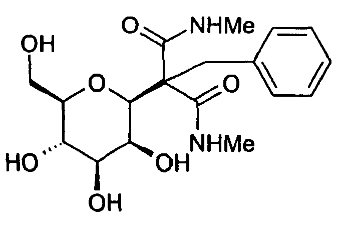 | 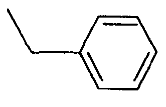 | 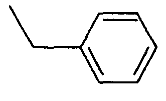 | -OH -CH2OH | 4 | -NHCH3 | -CH3 | 1 |
| B | 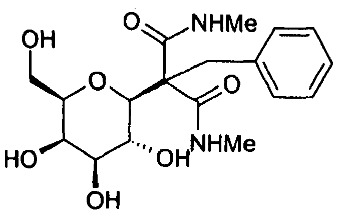 | 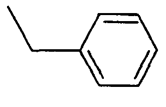 | 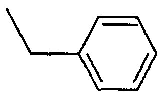 | -OH -CH2OH | 4 | -NHCH3 | -CH3 | 1 |
| C | 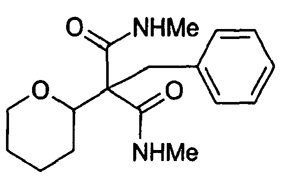 | 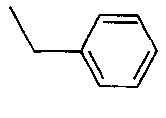 | 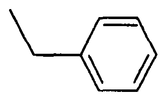 | -- | 0 | -NHCH3 | -CH3 | 1 |
| D | 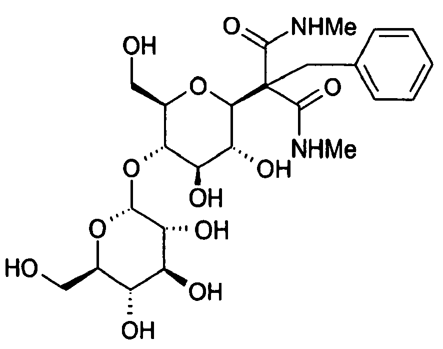 | 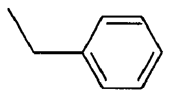 | 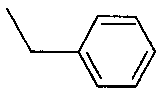 | -OH -CH2OH -sucre | 4 | -NHCH3 | -CH3 | 1 |
| E | 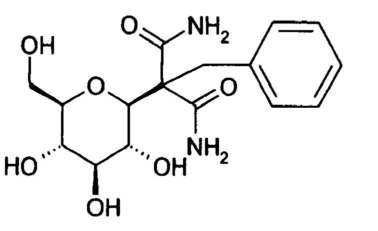 | 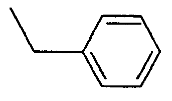 | 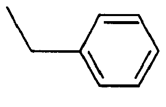 | -OH -CH2OH | 4 | -NH2 | H | 1 |
| F | 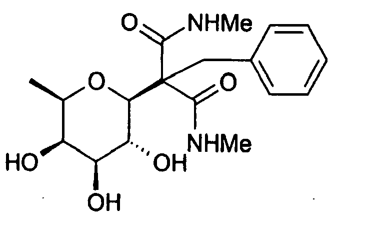 | 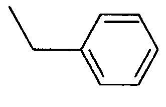 | 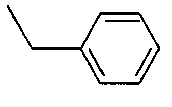 | -OH -CH3 | 4 | -NHCH3 | -CH3 | 1 |
| G | 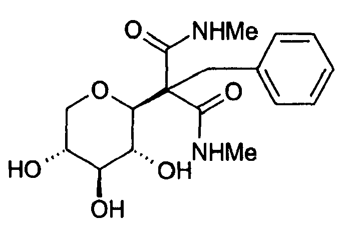 | 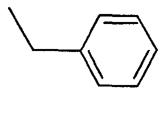 | 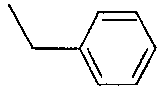 | -OH | 3 | -NHCH3 | -CH3 | 1 |
| H | 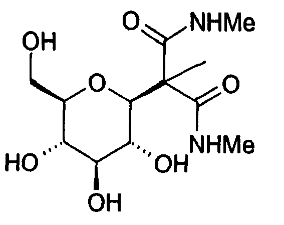 | -CH3 | -CH3 | -OH -CH2OH | 4 | -NHCH3 | -CH3 | 1 |
| I | 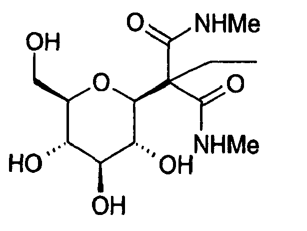 | -CH2-CH3 | -CH2-CH3 | -OH -CH2OH | 4 | -NHCH3 | -CH3 | 1 |
| J | 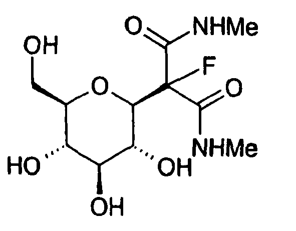 | F | F | -OH -CH2OH | 4 | -NHCH3 | -CH3 | 1 |
| K | 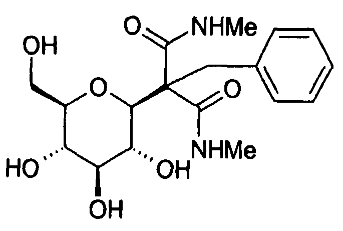 | 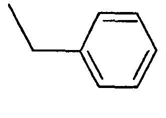 | 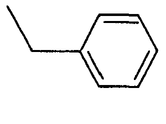 | -OH -CH2OH | 4 | -NHCH3 | -CH3 | 1 |
| L | 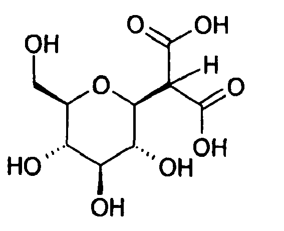 | H | H | -OH -CH2OH | 4 | -OH | - | 1 |

La composition selon l'invention contient un milieu physiologiquement acceptable et un ou plusieurs composés selon l'invention en une quantité efficace pour favoriser la desquamation de la peau, par exemple en une quantité comprise entre 0,01 et 30 % en poids et de préférence entre 0,1 et 5 % en poids, par rapport au poids total de la composition.

Par milieu physiologiquement acceptable, on comprend un milieu compatible avec la peau et éventuellement avec les muqueuses, les ongles, le cuir chevelu et/ou les cheveux.

La composition selon l'invention peut avoir la forme notamment d'une solution aqueuse ou d'une dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme shampooing ou après-shampooing.

Lorsque la composition utilisable selon l'invention est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le monostéarate de glycérol, le polysorbate 60 et les stéarates de polyéthylène glycol (20 OE, 40 OE, 100 OE).

De façon connue, la composition de l'invention peut contenir également les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, et les matières colorantes. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse, dans les vésicules lipidiques et/ou dans les nanoparticules. Ils seront choisis de manière à ne pas nuire aux propriétés desquamantes des composés selon l'invention.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe.

La composition selon l'invention peut être destinée à une application cosmétique ou pharmaceutique, particulièrement dermatologique. De préférence la composition selon l'invention est destinée à une application cosmétique.

L'invention a donc également pour objet un procédé de traitement cosmétique de la peau ou du cuir chevelu, comprenant l'application topique sur la peau ou le cuir chevelu de la composition décrite précédemment.

Compte tenu des propriétés desquamantes des composés selon l'invention, ce procédé est en particulier destiné à prévenir ou estomper les signes cutanés du vieillissement et/ou à améliorer l'éclat du teint et/ou à lutter contre la peau sèche.

L'invention a donc également pour objet un procédé cosmétique destiné à prévenir ou estomper les signes cutanés du vieillissement et/ou à améliorer l'éclat du teint et/ou à lutter contre la peau sèche, comprenant l'application topique sur la peau d'une composition telle que définie précédemment.

L'invention a encore pour objet l'utilisation de la composition définie précédemment pour la fabrication d'une préparation dermatologique destinée à traiter l'acné ou les hyperkératoses.

En outre, dans l'hypothèse où les propriétés desquamantes des composés selon l'invention seraient dues à leur capacité à se fixer sur le site actif des glycosidases impliquées dans le processus de desquamation, on pense que certains des composés selon l'invention pourraient inactiver certaines glycosidases épidermiques et présenter ainsi des propriétés anti-desquamantes conduisant à un épaississement du stratum corneum qui pourrait être mis à profit dans la protection de la peau contre les UV et les polluants.

La présente invention a donc également pour objet un procédé cosmétique de protection de la peau contre les méfaits des UV et de la pollution, comprenant l'application topique sur la peau de la composition selon l'invention.

Par ailleurs, les β-glycosidases endogènes sont impliquées dans la déglycosylation de précurseurs lipidiques tels que les glucosylcéramides en céramides, qui est l'une des étapes de maturation des lipides intercornéocytaires intervenant dans la fonction "barrière" de l'épiderme. En stimulant les β-glycosidases, les composés selon l'invention peuvent ainsi permettre de renforcer la fonction barrière de la peau. Cette propriété des composés selon l'invention permet en outre, en freinant l'évaporation de l'eau contenue dans la peau, de maintenir un bon taux d'hydratation cutanée.

La présente invention a donc encore pour objet un procédé cosmétique pour améliorer la fonction barrière de la peau et/ou hydrater la peau, comprenant l'application topique sur la peau de la composition selon l'invention.

D'autres caractéristiques et avantages de l'invention ressortiront mieux des exemples qui suivent, donnés à titre illustratif et non limitatif. Dans ce qui suit, les proportions sont données en pourcentage pondéral, sauf indication contraire.

### EXEMPLES

### Exemple 1 : Préparation du 2-benzyl-N,N'-diméthyl-2-(3,4,5-trihydroxy-6-méthyltétrahydro-pyran-2-yl)-malonamide

La Figure 1 illustre un procédé de préparation du 2-benzyl-N,N'-diméthyl-2-(3,4,5-trihydroxy-6-méthyl-tétrahydro-pyran-2-yl)-malonamide en trois étapes.

### Première Etape

Dans un ballon, on introduit le fucose (1 g ; 1 équivalent), le dérivé barbiturique (0,95 g ; 1,5 équivalent) et 20 ml d'eau. On ajoute de l'hydrogénocarbonate de sodium dans l'eau à pH 7 et on chauffe à 80°C jusqu'à disparition des produits de départ (environ 5 heures).
On évapore l'eau pour n'en garder qu'un volume minimal en vue de solubiliser le résidu obtenu. On fait précipiter le résidu dans du méthanol, puis on le filtre et sèche sous vide à l'aide de pentoxyde de diphosphore. On récupère 1,7 g d'un produit solide (rendement : 86%). La spectrométrie de masse et la RMN (400 MHz) sont conformes à la structure attendue.

### Deuxième étape

Dans un ballon, on introduit le composé issu de la première étape (0,2 g ; 1 équivalent), et le bromure de benzyle (0,1 ml ; 1,3 équivalent) dans 4 ml de DMF. On laisse agiter à température ambiante jusqu'à disparition des produits de départ (environ 5 heures).

On évapore le solvant, puis on procède à trois extractions au toluène suivies de 3 extractions à l'acétate d'éthyle. On sèche sur sulfate de sodium les phases d'acétate d'éthyle. Après évaporation, on obtient 0,15 g d'un produit huileux (rendement : 62%). La spectrométrie de masse et la RMN (400 MHz) sont conformes à la structure attendue.

### Troisième étape

Dans un ballon, on introduit le composé issu de la seconde étape (0,1 g ; 1 équivalent) dans 0,5 ml d'eau. On ajoute 0,5 ml (2 équivalents) de soude (1 M) et on laisse agiter à température ambiante pendant 30 mn. On ajoute ensuite l'acide acétique et on laisse encore agiter pendant une heure. On évapore le milieu réactionnel et on sèche, pour obtenir 0,17 g d'un produit pâteux (rendement : > 100% en raison de la présence d'acétate de sodium associé au produit). La spectrométrie de masse et la RMN (400 MHz) sont conformes à la structure attendue.

### Exemple 2 : Mise en évidence de l'effet desquamant des composés selon l'invention

Le test consiste à compter les cornéocytes libérés après incubation de lots de stratum corneum isolés en présence de trois composés testés, correspondant aux composés F, G et K du Tableau 1.

### Protocole :

Les composés F, G et K ont chacun été solubilisés à 2% en poids dans un tampon PBS 0,1% Triton X100 à pH 7,4.

On a utilisé deux échantillons différents de stratum corneum isolé par trypsine / chaleur à partir de plasties de chirurgie. Des disques de SCI de 4 mm de diamètre ont été découpés à l'emporte-pièce et disposés au fond d'une boîte de 96 puits. 50 µl de tampon contenant le composé à tester ont été ajoutés à chaque puits. Des témoins sans composé ont été préparés. Les essais ont été répétés trois fois. L'incubation a été réalisée à 37 °C sous agitation pendant 24 h. 10 µl ont alors été prélevés et disposés en cellule de Mallassez. Les cornéocytes libérés ont été comptés au microscope.

### Résultats :

Le Tableau 2 ci-après rassemble les résultats obtenus.

**Tableau 2**

| Moyenne du nombre de cornéocytes libérés | | |
|---|---|---|
| Composé testé | 1^{er} éch. de peau | 2ème éch. de peau |
| F | 5 | 12 |
| G | 13 | 12 |
| K | 7 | 5 |
| Témoin | 1 | 4 |

Ce test montre donc que les composés selon l'invention augmentent la quantité de cornéocytes libérée par le stratum corneum isolé, et sont donc utilisables comme desquamants.

### Exemple 3 : fluide anti-âge

| | |
|---|---|
| Composé F | 1 % |
| Octyldodécanol | 5 % |
| Huile de tournesol | 11 % |
| EDTA | 0,05 % |
| Gomme de xanthane | 0,2 % |
| Polyacrylamide/isoparaffine/Laureth-7 (Sepigel 305 vendu par la société Seppic) | 0,9 % |
| Cyclopentasiloxane | 5 % |
| Glycérine | 4 % |
| Polyacrylate de glycéryle à 2 % dans un mélange | |
| eau/glycérine (Lubrajel vendu par la société Guardian) | 5 % |
| Stéarate de glycérol | 0,6 % |
| Stéarate de polyoxyéthylène (100 OE) | 0,6 % |
| Stéarate de polyoxyéthylène (20 OE) | 1,2 % |
| Acide stéarique | 0,6 % |
| Alcool stéarylique | 1 % |
| Conservateurs | 0,3 % |
| Eau | qsp 100 % |

Ce fluide, appliqué matin et soir, permet d'atténuer les signes du vieillissement cutané et d'améliorer l'éclat du teint.

### Exemple 4 : gel anti-acnéique

| | |
|---|---|
| Composé E | 3,0 % |
| Ethanol à 90° | 50,0 % |
| Propylène glycol | 45,5 % |
| Hydroxypropylcellulose | 1,5 % |

### Exemple 5 :crème hydratante

| | |
|---|---|
| Composé I | 6,0 % |
| Monostéarate de glycérol | 0,8 % |
| Alcool cétylique | 2,0 % |
| Alcool stéarylique | 5,0 % |
| Stéarate de polyoxyéthylène (20 OE) | 3,0 % |
| Acide acrylique réticulé (CARBOPOL 941) | 0,3 % |
| Triglycérides capryliques / capriques | 12,0 % |
| Conservateurs | qs |
| Eau | qsp 100 % |

## Revendications

1. Composition adaptée à une application topique sur la peau ou le cuir chevelu contenant, dans un milieu physiologiquement acceptable comprenant un adjuvant cosmétique ou dermatologique, au moins un composé répondant à la formule (I) suivante : 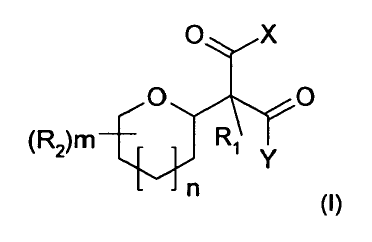 dans laquelle:
R₁ représente un atome d'hydrogène, ou un groupement choisi parmi :
- un groupe alkyle en C₁-C₁₂, saturé ou insaturé, linéaire, cyclique ou ramifié, éventuellement substitué par un ou plusieurs groupes choisis parmi -OR, -SR, - COOR, -NRR', halogène, sulfate, phosphate, glycoside, aryle et hétérocycle,
où R et R' représentent, indépendamment l'un de l'autre, un atome d'hydrogène, ou un groupe alkyle en C₁-C₁₂, saturé ou insaturé, linéaire, cyclique ou ramifié,
- un atome d'halogène,
- un groupe aryle éventuellement substitué par un ou plusieurs groupes choisis parmi :
- OR, -SR, -COOR, -NRR', halogène, sulfate et phosphate où R et R' ont la signification indiquée précédemment;
R₂ représente un groupement choisi parmi :
- R₂₁ où R₂₁ a la définition donnée ci-dessus pour R₁,
- OR₂₂, où R₂₂ a la définition donnée ci-dessus pour R₁ excepté halogène,
- OR₂₃ où R₂₃ est un groupe sulfate, phosphate, glycoside, alkylcarbonyle ou un hétérocycle,
- NR₂₄R₂₅ où R₂₄ et R₂₅ représentent indépendamment un groupe ayant l'une des défintions données ci-dessus pour R₁, excepté halogène,
- NR₂₆R₂₇ où R₂₆ et R₂₇ représentent indépendamment un radical glycoside ou alkylcarbonyle ou un hétérocycle,
- un groupe sulfate ou phosphate ;
X et Y représentent indépendamment l'un de l'autre un radical -OR₃ ou -NR₃R₄, où R₃ et R₄ sont indépendamment choisis parmi :
- un atome d'hydrogène,
- un groupe alkyle en C₁-C₁₂, saturé ou insaturé, linéaire, cyclique ou ramifié, éventuellement substitué par un ou plusieurs groupes choisis parmi -OR, -SR, - COOR, -NRR', halogène, sulfate, phosphate, glycoside, aryle et hétérocycle où R et R' ont la signification indiquée précédemment,
- un groupe aryle éventuellement substitué par un ou plusieurs groupes choisis parmi :
- OR, -SR, -COOR, -NRR', halogène, sulfate et phosphate, où R et R' ont la signification indiquée précédemment,
- ou R₃ et R₄ forment ensemble un cycle comportant de 5 à 7 atomes avec l'atome d'azote auquel ils sont reliés,
ou X et Y forment un cycle de 6 à 7 atomes de carbone avec les trois atomes de carbone les séparant ;
n est un entier égal à 0 ou 1 ; et
m est un entier compris entre 0 et 4.

2. Composition selon la revendication 1, **caractérisée en ce que**
- R₁ est un atome de fluor ou d'hydrogène ou un radical alkyle non substitué ou benzyle,
- R₂ est un groupe hydroxyle, hydroxyalkyle ou alkyle ou un reste de sucre,
- X et Y sont des groupes -NR₃R₄ où R₃ et R₄ sont indépendamment choisis parmi un atome d'hydrogène ; et un radical méthyle, éthyle, n-propyle ou isopropyle, et
- n est égal à 1.

3. Composition adaptée à une application topique sur la peau ou le cuir chevelu contenant, dans un milieu physiologiquement acceptable, au moins un composé répondant à la formule (II) suivante : 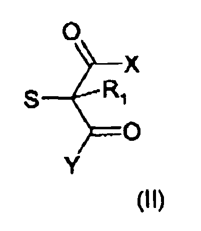 dans laquelle :
- S représente un monosaccharide ou un polysaccharide comportant jusqu'à 20 unités sucre, sous forme pyranose et/ou furanose et de série L et/ou D, ledit mono- ou polysaccharide présentant au moins une fonction hydroxyle libre,
- la liaison S-C- représente une liaison de nature C-anomérique,
- R₁ représente un atome d'hydrogène, ou un groupement choisi parmi:
- un groupe alkyle en C₁-C₁₂, saturé ou insaturé, linéaire, cyclique ou ramifié, éventuellement substitué par un ou plusieurs groupes choisis parmi -OR, -SR, -COOR, -NRR', halogène, sulfate, phosphate, glycoside, aryle et hétérocycle, où R et R' représentent, indépendamment l'un de l'autre, un atome d'hydrogène, ou un groupe alkyle en C₁-C₁₂, saturé ou insaturé, linéaire, cyclique ou ramifié,
- un atome d'halogène,
- un groupe aryle éventuellement substitué par un ou plusieurs groupes choisis parmi : -OR, -SR, -COOR, -NRR', halogène, sulfate et phosphate où R et R' ont la signification indiquée précédemment;
- X et Y représentent indépendamment l'un de l'autre un radical -OR₃ ou -NR₃R₄, où R₃ et R₄ sont indépendamment choisis parmi :
- un atome d'hydrogène,
- un groupe alkyle en C₁-C₁₂, saturé ou insaturé, linéaire, cyclique ou ramifié, éventuellement substitué par un ou plusieurs groupes choisis parmi -OR, -SR, -COOR, -NRR', halogène, sulfate, phosphate, glycoside, aryle et hétérocycle où R et R' ont la signification indiquée précédemment,
- un groupe aryle éventuellement substitué par un ou plusieurs groupes choisis parmi : -OR, -SR, -COOR, -NRR', halogène, sulfate et phosphate, où R et R' ont la signification indiquée précédemment,
- ou R₃ et R₄ forment ensemble un cycle comportant de 5 à 7 atomes avec l'atome d'azote auquel ils sont reliés,
ou X et Y forment un cycle de 6 à 7 atomes de carbone avec les trois atomes de carbone les séparant.

4. Composition selon la revendication précédente, **caractérisée par le fait qu'**elle contient au moins un composé répondant à la formule (III) suivante : 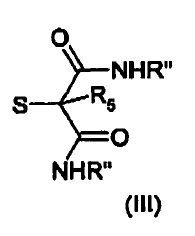 dans laquelle :
- S représente un monosaccharide ou un polysaccharide comportant jusqu'à 20 unités sucre, sous forme pyranose et/ou furanose et de série L et/ou D, ledit mono- ou polysaccharide présentant au moins une fonction hydroxyle libre,
- la liaison S-C- représente une liaison de nature C-anomérique,
- R₅ désigne :
un groupe alkyle en C₁-C₁₂ saturé ou insaturé, linéaire, cyclique ou ramifié, non substitué, ou
un radical benzyle, ou
un atome d'halogène ;
- R" désigne un atome d'hydrogène ou un groupe alkyle en C₁-C₁₂, saturé ou insaturé, linéaire, cyclique ou ramifié, non substitué.

5. Composition selon la revendication 3 ou 4, **caractérisée en ce que** le monosaccharide est choisi parmi le D-glucose, le D-galactose, le D-mannose, le D-xylose, le D-lyxose, le L-fucose, L-arabinose, le L-rhamnose, l'acide D-glucuronique, l'acide D-galacturonique, l'acide D-iduronique, la N-acétyl-D-glucosamine, et la N-acétyl- D-galactosamine.

6. Composition selon l'une des revendications 1 ou 2, **caractérisé en ce que** le composé de formule (I) est choisi parmi les composés suivants :

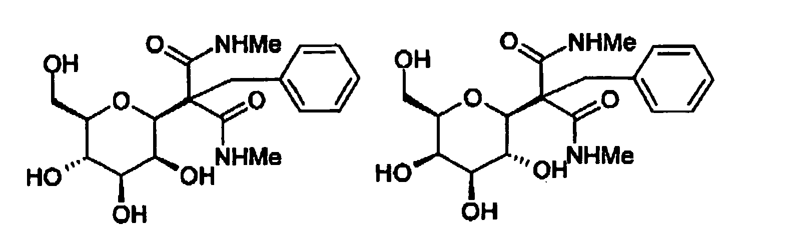

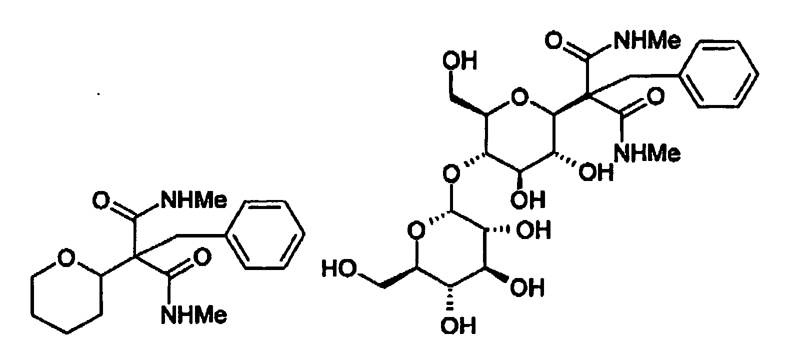

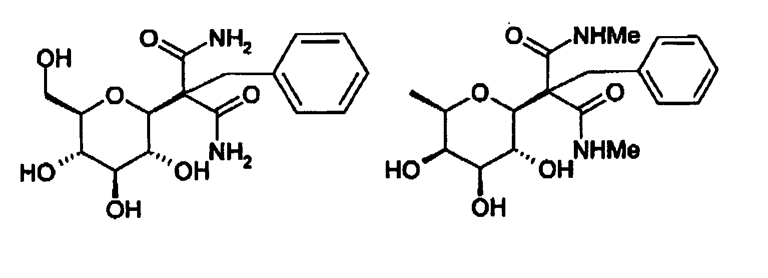

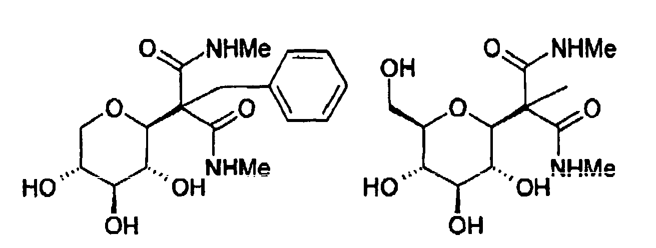

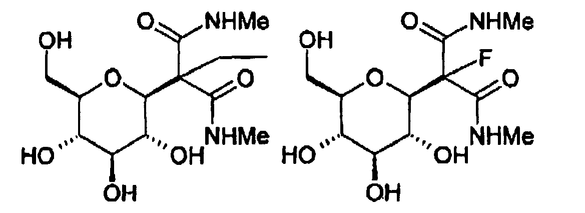

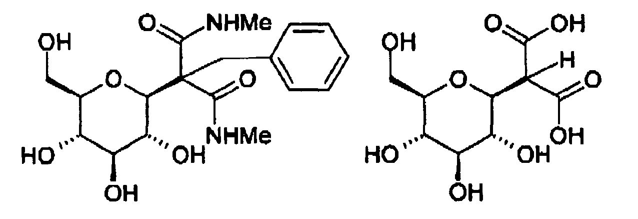

7. Composition selon la revendication3 ou 4, **caractérisée en ce que** le polysaccharide contenant jusqu'à 6 unités sucre est choisi parmi le D-maltose, le D-lactose, le D-cellobiose, le D-mattotriose, un disaccharide associant un acide uronique choisi parmi l'acide D-iduronique ou l'acide D-glucuronique avec une hexosamine choisi parmi la D-galactosamine, la D-glucosamine, la N-acétyl-D-galactosamine, la N-acétyl-D-glucosamine, un oligosaccharide contenant au moins un xylose avantageusement choisis parmi le xyloblose, le méthyl-β-xylobioside, le xylotriose, le xyotétraose, le xylopentaose.

8. Composition selon l'une quelconque des revendications 4 à 7, **caractérisée en ce que** R₅ est un groupe benzyle ou méthyle et R" est un groupe méthyle.

9. Dérivé C-glycoside répondant à la formule (III) : 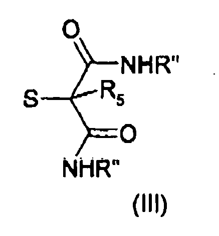 dans laquelle :
- S représente un monosaccharide ou un polysaccharide comportant jusqu'à 20 unités sucre, sous forme pyranose et/ou furanose et de série L et/ou D, ledit mono- ou polysaccharide présentant au moins une fonction hydroxyle libre,
- la liaison S-C- représente une liaison de nature C-anomérique,
- R₅ désigne :
un groupe alkyle en C₁-C₁₂ saturé ou Insaturé, linéaire, cyclique ou ramifié, non substitué, ou
un radical benzyle, ou
un atome d'halogène ;
- R" désigne un atome d'hydrogène ou un groupe alkyle en C₁-C₁₂, saturé ou insaturé, linéaire, cyclique ou ramifié, non substitué.

10. Dérivé C-glycoside selon la revendication 9, **caractérisé en ce qu'**il est choisi parmi les composés suivants :

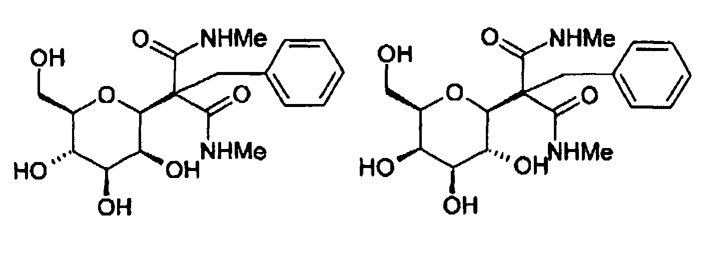

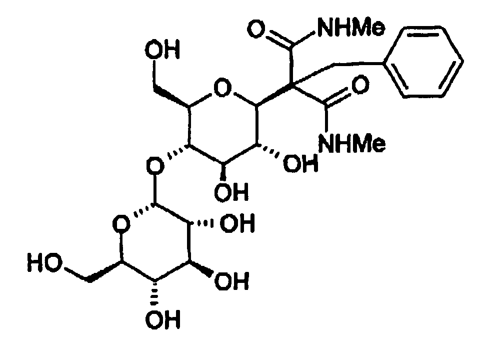

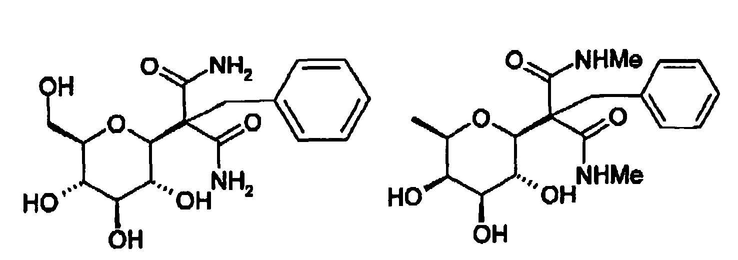

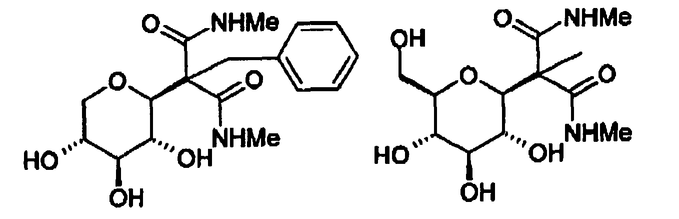

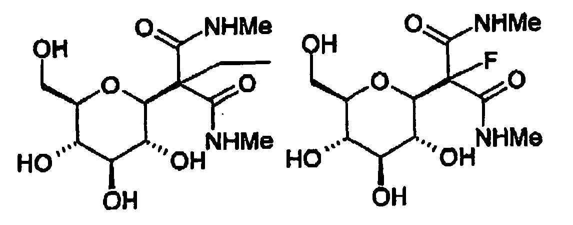

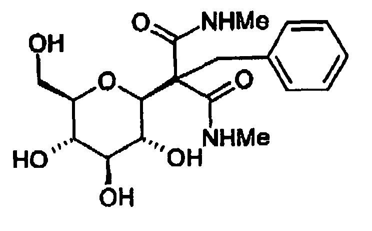

11. Dérivé C-glycoside selon la revendication 9 ou 10, **caractérisé en ce que** R₅ est un groupe benzyle ou méthyle et R" est un groupe méthyle.

12. Procédé de traitement cosmétique non thérapeutique de la peau ou du cuir chevelu, comprenant l'application topique sur la peau ou le cuir chevelu d'une composition telle que définie dans l'une quelconque des revendications 1 à 8.

13. Procédé cosmétique non thérapeutique destiné à prévenir ou estomper les signes cutanés du vieillissement et/ou à améliorer l'éclat du teint, et/ou lutter contre la peau sèche comprenant l'application topique sur la peau d'une composition telle que définie dans l'une quelconque des revendications 1 à 8.

14. Utilisation de la composition définie dans l'une quelconque des revendications 1 à 8 pour la fabrication d'une préparation dermatologique destinée à traiter l'acné ou les hyperkératoses.

15. Procédé cosmétique non thérapeutique de protection de la peau contre les méfaits des UV et de la pollution, comprenant l'application topique sur la peau d'une composition selon l'une quelconque des revendications 1 à 8.

16. Procédé cosmétique non thérapeutique pour améliorer la fonction barrière de la peau et/ou hydrater la peau, comprenant l'application topique sur la peau d'une composition selon l'une quelconque des revendications 1 à 8.

## Claims

1. Composition that is suitable for topical application to the skin or the scalp, containing, in a physiologically acceptable medium comprising a cosmetic or dermatological adjuvant, at least one compound corresponding to formula (I) below: 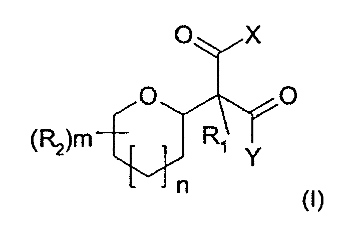 in which:
R₁ represents a hydrogen atom or a group chosen from:
- a saturated or unsaturated, linear, cyclic or branched C₁-C₁₂ alkyl group, optionally substituted with one or more groups chosen from -OR, -SR, -COOR, -NRR', halogen, sulfate, phosphate, glycoside, aryl and heterocycle,
in which R and R' represent, independently of each other, a hydrogen atom or a saturated or unsaturated, linear, cyclic or branched C₁-C₁₂ alkyl group,
- a halogen atom,
- an aryl group optionally substituted with one or more groups chosen from:
-OR, -SR, -COOR, -NRR', halogen, sulfate and phosphate, in which R and R' have the meaning given above;
R₂ represents a group chosen from:
- R₂₁ in which R₂₁ has the definition given above for R₁,
- OR₂₂, in which R₂₂ has the definition given above for R₁, with the exception of halogen,
- OR₂₃, in which R₂₃ is a sulfate, phosphate, glycoside or alkylcarbonyl group, or a heterocycle,
- NR₂₄R₂₅, in which R₂₄ and R₂₅ independently represent a group having one of the definitions given above for R₁, with the exception of halogen,
- NR₂₆R₂₇, in which R₂₆ and R₂₇ independently represent a glycoside or alkylcarbonyl radical or a heterocycle,
- a sulfate or phosphate group;
X and Y represent, independently of each other, a radical -OR₃ or -NR₃R₄, in which R₃ and R₄ are independently chosen from:
- a hydrogen atom,
- a saturated or unsaturated, linear, cyclic or branched C₁-C₁₂ alkyl group, optionally substituted with one or more groups chosen from -OR, -SR, -COOR, -NRR', halogen, sulfate, phosphate, glycoside, aryl and heterocycle, in which R and R' have the meaning given above,
- an aryl group optionally substituted with one or more groups chosen from: -OR, -SR, -COOR, -NRR', halogen, sulfate and phosphate, in which R and R' have the meaning given above,
- or R₃ and R₄ together form a ring containing from 5 to 7 atoms with the nitrogen atom to which they are attached,
or X and Y form a ring of 6 or 7 carbon atoms with the three carbon atoms separating them;
n is an integer equal to 0 or 1; and
m is an integer between 0 and 4.

2. Composition according to Claim 1, **characterized in that**
- R₁ is a fluorine or hydrogen atom or an unsubstituted alkyl or benzyl radical,
- R₂ is a hydroxyl, hydroxyalkyl or alkyl group or a sugar residue,
- X and Y are groups -NR₃R₄ in which R₃ and R₄ are chosen independently from a hydrogen atom and a methyl, ethyl, n-propyl or isopropyl radical, and
- n is equal to 1.

3. Composition that is suitable for topical application to the skin or the scalp, containing, in a physiologically acceptable medium, at least one compound corresponding to formula (II) below: 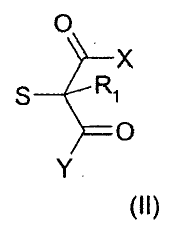 in which:
- S represents a monosaccharide or a polysaccharide containing up to 20 sugar units, in pyranose and/or furanose form and of L and/or D series, the said monosaccharide or polysaccharide containing at least one free hydroxyl function,
- the S-C bond represents a bond of C-anomeric nature,
- R₁ represents a hydrogen atom or a group chosen from:
- a saturated or unsaturated, linear, cyclic or branched C₁-C₁₂ alkyl group, optionally substituted with one or more groups chosen from -OR, -SR, -COOR, -NRR', halogen, sulfate, phosphate, glycoside, aryl and heterocycle,
in which R and R' represent, independently of each other, a hydrogen atom or a saturated or unsaturated, linear, cyclic or branched C₁-C₁₂ alkyl group,
- a halogen atom,
- an aryl group optionally substituted with one or more groups chosen from: -OR, -SR, -COOR, -NRR', halogen, sulfate and phosphate, in which R and R' have the meaning given above;
- X and Y represent, independently of each other, a radical -OR₃ or -NR₃R₄, in which R₃ and R₄ are independently chosen from:
- a hydrogen atom,
- a saturated or unsaturated, linear, cyclic or branched C₁-C₁₂ alkyl group, optionally substituted with one or more groups chosen from -OR, -SR,
- COOR, -NRR', halogen, sulfate, phosphate, glycoside, aryl and heterocycle, in which R and R' have the meaning given above,
- an aryl group optionally substituted with one or more groups chosen from: -OR, -SR, -COOR, -NRR', halogen, sulfate and phosphate, in which R and R' have the meaning given above,
- or R₃ and R₄ together form a ring containing from 5 to 7 atoms with the nitrogen atom to which they are attached,
or X and Y form a ring of 6 or 7 carbon atoms with the three carbon atoms separating them.

4. Composition according to the preceding claim, **characterized in that** it contains at least one compound corresponding to formula (III) below: 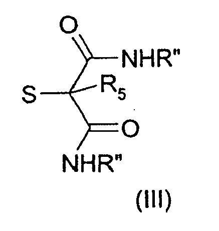 in which:
- S represents a monosaccharide or a polysaccharide containing up to 20 sugar units, in pyranose and/or furanose form and of L and/or D series, the said monosaccharide or polysaccharide containing at least one free hydroxyl function,
- the S-C bond represents a bond of C-anomeric nature,
- R₅ denotes:
a saturated or unsaturated, linear, cyclic or branched, unsubstituted C₁-C₁₂ alkyl group, or
a benzyl radical, or
a halogen atom;
- R" denotes a hydrogen atom or a saturated or unsaturated, linear, cyclic or branched, unsubstituted C₁-C₁₂ alkyl group.

5. Composition according to Claim 3 or 4, **characterized in that** the monosaccharide is chosen from D-glucose, D-galactose, D-mannose, D-xylose, D-lyxose, L-fucose, L-arabinose, L-rhamnose, D-glucuronic acid, D-galacturonic acid, D-iduronic acid, N-acetyl-D-glucosamine and N-acetyl-D-galactosamine.

6. Composition according to either of Claims 1 and 2, **characterized in that** the compound of formula (I) is chosen from the following compounds:

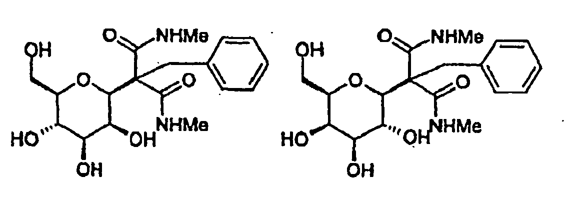

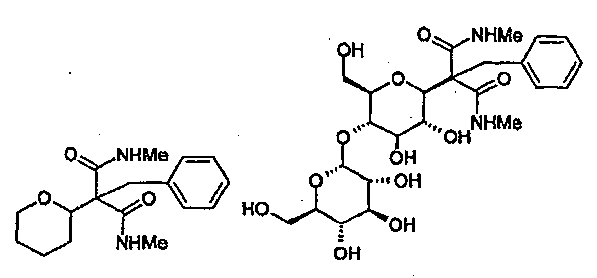

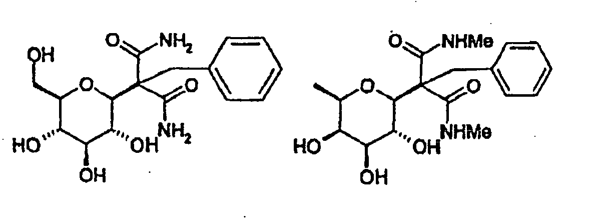

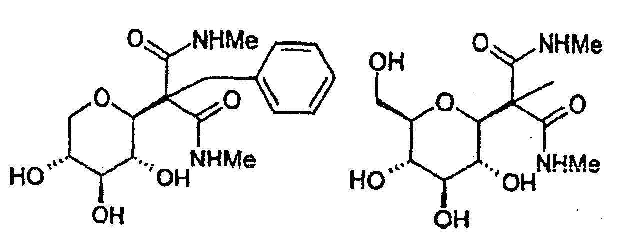

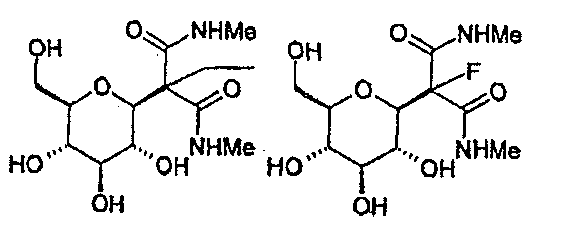

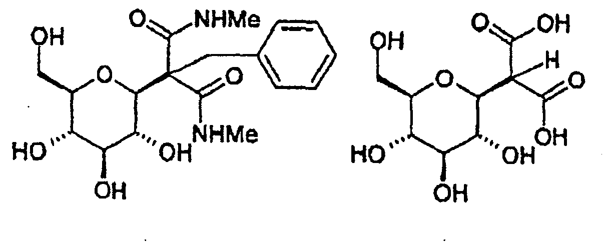

7. Composition according to Claim 3 or 4, **characterized in that** the polysaccharide containing up to 6 sugar units is chosen from D-maltose, D-lactose, D-cellobiose, D-maltotriose, a disaccharide combining a uronic acid chosen from D-iduronic acid and D-glucuronic acid with a hexosamine chosen from D-galactosamine, D-glucosamine, N-acetyl-D-galactosamine, N-acetyl-D-glucosamine, an oligosaccharide containing at least one xylose advantageously chosen from xylobiose, methyl-β-xylobioside, xylotriose, xylotetraose and xylopentaose.

8. Composition according to any one of Claims 4 to 7, **characterized in that** R₅ is a benzyl or methyl group and R" is a methyl group.

9. C-Glycoside derivative corresponding to formula (III) : 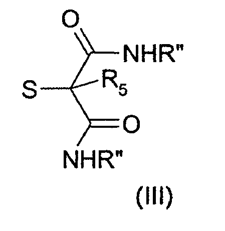 in which:
- S represents a monosaccharide or a polysaccharide containing up to 20 sugar units, in pyranose and/or furanose form and of L and/or D series, the said monosaccharide or polysaccharide containing at least one free hydroxyl function,
- the S-C bond represents a bond of C-anomeric nature,
- R₅ denotes:
a saturated or unsaturated, linear, cyclic or branched, unsubstituted C₁-C₁₂ alkyl group, or
a benzyl radical, or
a halogen atom;
- R" denotes a hydrogen atom or a saturated or unsaturated, linear, cyclic or branched, unsubstituted C₁-C₁₂ alkyl group.

10. C-Glycoside derivative according to Claim 9, **characterized in that** it is chosen from the following compounds:

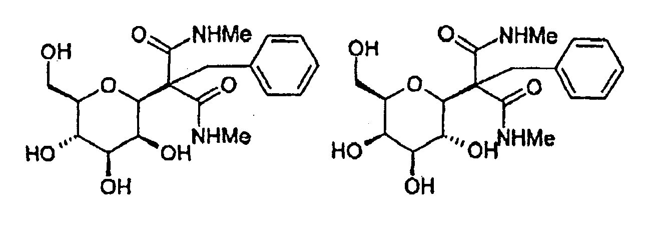

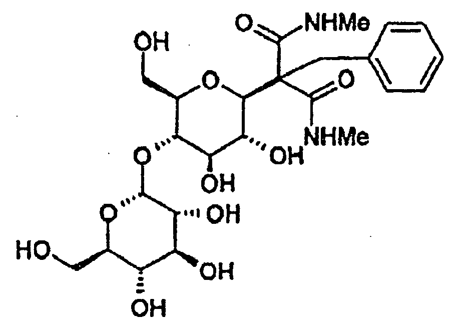

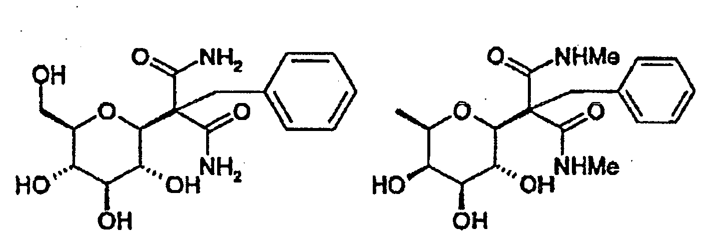

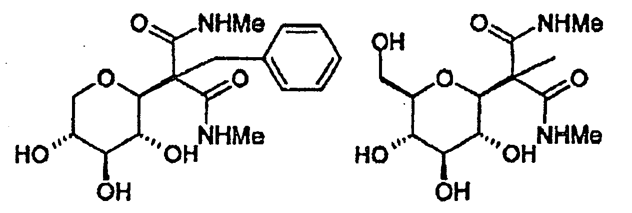

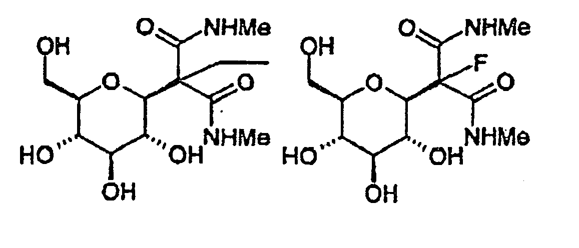

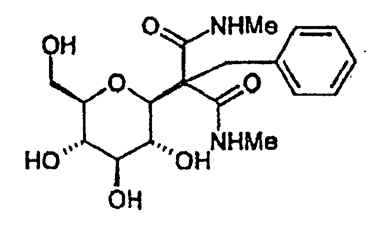

11. C-Glycoside derivative according to Claim 9 or 10, **characterized in that** R₅ is a benzyl or methyl group and R" is a methyl group.

12. Non-therapeutic cosmetic process for treating the skin or the scalp, comprising the topical application to the skin or the scalp of a composition as defined in any one of Claims 1 to 8.

13. Non-therapeutic cosmetic process for preventing or fading out the signs of ageing of the skin and/or for improving the radiance of the complexion and/or for combating dry skin, comprising the topical application to the skin of a composition as defined in any one of Claims 1 to 8.

14. Use of the composition defined in any one of Claims 1 to 8, for the manufacture of a dermatological preparation for treating acne or hyperkeratosis.

15. Non-therapeutic cosmetic process for protecting the skin against the harmful effects of UV rays and pollution, comprising the topical application to the skin of a composition according to any one of Claims 1 to 8.

16. Non-therapeutic cosmetic process for improving the barrier function of the skin and/or for moisturizing the skin, comprising the topical application to the skin of a composition according to any one of Claims 1 to 8.

## Patentansprüche

1. Für eine topische Anwendung auf der Haut oder der Kopfhaut geeignete Zusammensetzung, die in einem physiologisch geeigneten Medium, das einen kosmetischen oder dermatologischen Hilfsstoff enthält, mindestens eine Verbindung der folgenden Formel (I) enthält, 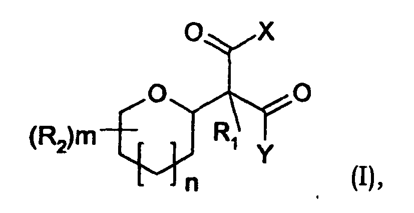 worin bedeuten:
R₁ ein Wasserstoffatom oder eine Gruppe, die ausgewählt ist unter:
- einer gesättigten oder ungesättigten, geradkettigen, cyclischen oder verzweigten C₁-C₁₂-Alkylgruppe, die gegebenenfalls mit einer oder mehreren Gruppen substituiert ist, die ausgewählt sind unter -OR, -SR, -COOR, -NRR', Halogen, Sulfat, Phosphat, Glycosid, Aryl und einer heterocyclischen Gruppe, wobei R und R' unabhängig voneinander ein Wasserstoffatom oder eine gesättigte oder ungesättigte, geradkettige, cyclische oder verzweigte C₁-C₁₂-Alkylgruppe bedeuten,
- einem Halogenatom,
- einer Arylgruppe, die gegebenenfalls mit einer oder mehreren Gruppen substituiert ist, die ausgewählt sind unter:
-OR, -SR, -COOR, -NRR', Halogen, Sulfat und Phosphat, wobei R und R' die oben angegebene Bedeutung besitzen;
R₂ eine Gruppe, die ausgewählt ist unter:
- R₂₁, wobei R₂₁ wie oben R₁ definiert ist,
- -OR₂₂, wobei R₂₂ wie oben R₁ definiert ist, mit Ausnahme von Halogen,
- -OR₂₃, wobei R₂₃ eine Sulfatgruppe, eine Phosphatgruppe, eine Glycosidgruppe, eine Alkylcarbonylgruppe oder eine heterocyclische Gruppe ist,
- NR₂₄R₂₅, wobei R₂₄ und R₂₅ unabhängig eine Gruppe bedeuten, wie sie oben für R₁ definiert wurde, mit Ausnahme von Halogen,
- NR₂₆R₂₇, wobei R₂₆ und R₂₇ unabhängig eine Glycosidgruppe oder eine Alkylcarbonylgruppe oder einen Heterocyclus bedeuten,
- einer Sulfatgruppe oder einer Phosphatgruppe,
X und Y unabhängig voneinander eine Gruppe -OR₃ oder -NR₃R₄, wobei R₃ und R₄ unabhängig ausgewählt sind unter:
- einem Wasserstoffatom,
- einer gesättigten oder ungesättigten, geradkettigen, cyclischen oder verzweigten C₁-C₁₂-Alkylgruppe, die gegebenenfalls mit einer oder mehreren Gruppen substituiert ist, die ausgewählt sind unter -OR, -SR, -COOR, -NRR', Halogen, Sulfat, Phosphat, Glycosid, Aryl und einer heterocyclischen Gruppe, wobei R und R' die oben angegebene Bedeutung besitzen,
- einer Arylgruppe, die gegebenenfalls mit einer oder mehreren Gruppen substituiert ist, die ausgewählt sind unter -OR, -SR, -COOR, -NRR', Halogen, Sulfat und Phosphat, wobei R und R' die oben angegeben Bedeutung besitzen,
- oder R₃ und R₄ bilden zusammen mit dem Stickstoffatom, an dem sie gebunden sind, einen Ring mit 5 bis 7 Atomen,
oder X und Y bilden mit den drei Kohlenstoffatomen, die sie trennen, einen Ring mit 6 bis 7 Kohlenstoffatomen,
n eine ganze Zahl gleich 0 oder 1 und
m eine ganze Zahl von 0 bis 4.

2. Zusammensetzung nach Anspruch 1, **dadurch gekennzeichnet, dass**
- R₁ ein Fluoratom oder ein Wasserstoffatom oder eine nicht-substituierte Alkylgruppe oder Benzyl ist,
- R₂ eine Hydroxygruppe, eine Hydroxyalkylgruppe oder eine Alkylgruppe oder ein Zuckerrest ist,
- X und Y Gruppen NR₃R₄ sind, wobei R₃ und R₄ unabhängig ausgewählt sind unter einem Wasserstoffatom und einer Methylgruppe, Ethylgruppe, n-Propylgruppe oder Isopropylgruppe,
und
- n gleich 1 ist.

3. Für eine topische Anwendung auf der Haut oder der Kopfhaut geeignete Zusammensetzung, die in einem physiologisch akzeptablen Medium mindestens eine Verbindung der folgenden **Formel (II) enthält:** 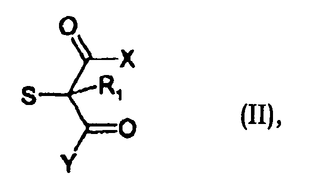 worin bedeuten:
- S ein Monosaccharid oder ein Polysaccharid mit bis zu 20 Zuckereinheiten in Pyranose- und/oder Furanose-Form sowie in L- und/oder D-Form, wobei das Monosaccharid oder Polysaccharid mindestens eine freie Hydroxylfunktion aufweist,
- die Bindung S-C- eine C-Anomer-Bindung,
- R₁ ein Wasserstoffatom oder eine Gruppe, die ausgewählt ist unter:
- einer gesättigten oder ungesättigten, geradkettigen, cyclischen oder verzweigten C₁-C₁₂-Alkylgruppe, die gegebenenfalls mit einer oder mehreren Gruppen substituiert ist, die ausgewählt sind unter -OR, -SR, -COOR,
- NRR', Halogen, Sulfat, Phosphat, Glycosid, Aryl und einer heterocyclischen Gruppe,
wobei R und R' unabhängig voneinander ein Wasserstoffatom oder eine gesättigte oder ungesättigte, geradkettige, cyclische oder verzweigte C₁-C₁₂-Alkylgruppe bedeuten,
- einem Halogenatom,
- einer Arylgruppe, die gegebenenfalls mit einer oder mehreren Gruppen substituiert ist, die ausgewählt sind unter -OR, -SR, -COOR, -NRR', Halogen, Sulfat und Phosphat, wobei R und R' die oben angegebene Bedeutung besitzen;
- X und Y unabhängig voneinander eine Gruppe -OR₃ oder -NR₃R₄, wobei R₃ und R₄ unabhängig ausgewählt sind unter:
- einem Wasserstoffatom,
- einer gesättigten oder ungesättigten, geradkettigen, cyclischen oder verzweigten C₁-C₁₂-Alkylgruppe, die gegebenenfalls mit einer oder mehreren Gruppen substituiert ist, die ausgewählt sind unter -OR, -SR, -COOR,
- NRR', Halogen, Sulfat, Phosphat, Glycosid, Aryl und einer heterocyclischen Gruppe, wobei R und R' die oben angegebene Bedeutung besitzen,
- einer Arylgruppe, die gegebenenfalls mit einer oder mehreren Gruppen substituiert ist, die ausgewählt sind unter -OR, -SR, -COOR, -NRR', Halogen, Sulfat und Phosphat, wobei R und R' die oben angegebene Bedeutung besitzen,
- oder R₃ und R₄ bilden zusammen mit dem Stickstoffatom, an dem sie gebunden sind, einen Ring mit 5 bis 7 Atomen,
oder X und Y bilden mit den drei Kohlenstoffatomen, die sie trennen, einen Ring mit 6 bis 7 Kohlenstoffatomen.

4. Zusammensetzung nach dem vorhergehenden Anspruch, **dadurch gekennzeichnet, dass** sie mindestens eine Verbindung der folgenden Formel (III) enthält: 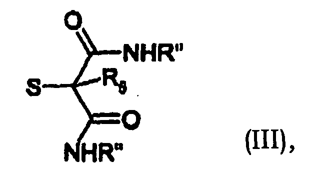 worin bedeuten:
- S ein Monosaccharid oder ein Polysaccharid mit bis zu 20 Zuckereinheiten in Pyranose- und/oder Furanose-Form sowie in L- und/oder D-Form, wobei das Monosaccharid oder Polysaccharid mindestens eine freie Hydroxylfunktion aufweist,
- die Bindung S-C- eine C-Anomer-Bindung,
- R₅ :
eine gesättigte oder ungesättigte, geradkettige, cyclische oder verzweigte, unsubstituierte C₁-C₁₂-Alkylgruppe oder
eine Benzylgruppe oder
ein Halogenatom,
- R" ein Wasserstoffatom oder eine gesättigte oder ungesättigte, geradkettige, cyclische oder verzweigte, nicht-substituierte C₁-C₁₂ Alkylgruppe.

5. Zusammensetzung nach Anspruch 3 oder 4, **dadurch gekennzeichnet, dass** das Monosaccharid ausgewählt ist unter D-Glucose, D-Galactose, D-Mannose, D-Xylose, D-Lyxose, D-Fucose, L-Arabinose, L-Rhamnose, D-Glucuronsäure, D-Galacturonsäure, D-Iduronsäure, N-Acetyl-D-glucosamin und N-Acetyl-D-galactosamin.

6. Zusammensetzung nach Anspruch 1 oder 2, **dadurch gekennzeichnet, dass** die Verbindung der Formel (I) unter folgenden Verbindungen ausgewählt ist:

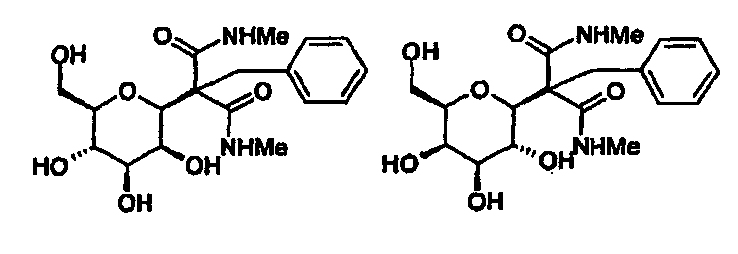

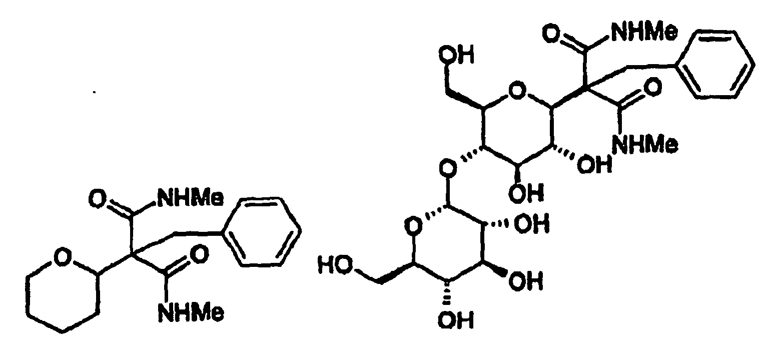

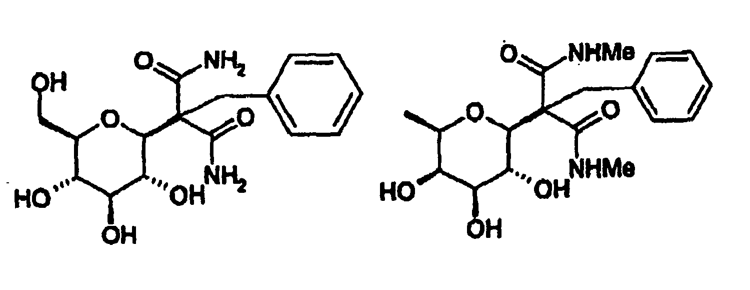

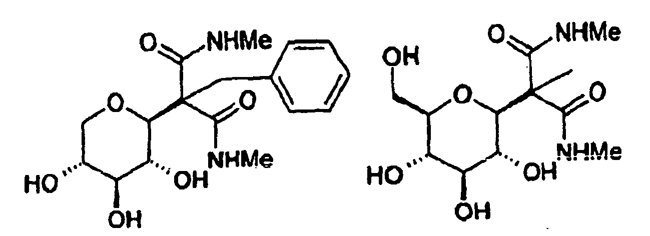

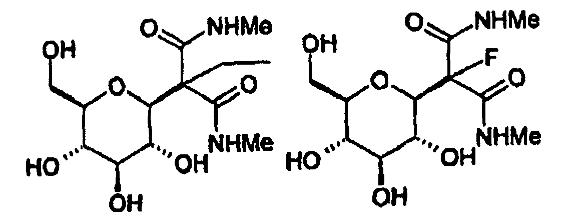

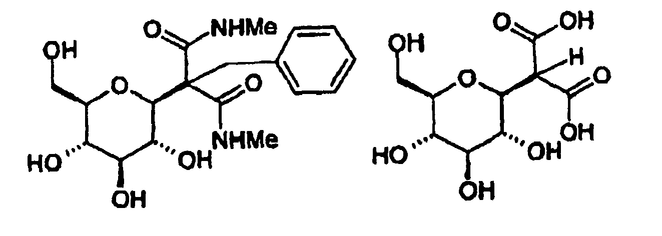

7. Zusammensetzung nach Anspruch 3 oder 4, **dadurch gekennzeichnet, dass** das Polysaccharid, das bis zu 6 Zuckereinheiten enthält, ausgewählt ist und D-Maltose, D-Lactose, D-Cellobiose, D-Maltotriose, einem Disaccharid, bei dem eine Uronsäure, die unter D-Iduronsäure oder D-Glucuronsäure ausgewählt ist, mit einem Hexosamin kombiniert ist, das ausgewählt ist unter D-Galactosamin, D-Glucosamin, N-Acetyl-D-galactosamin, N-Acetyl-D-glucosamin, einem Oligosaccharid, das mindestens eine Xylose enthält, die vorteilhaft ausgewählt ist unter Xylobiose, Methyl-β-xylobiosid, Xylotriose, Xylotetrose und Xylopentose.

8. Zusammensetzung nach einem der Ansprüche 4 bis 7, **dadurch gekennzeichnet, dass** R₅ eine Benzylgruppe, eine Methylgruppe und R" eine Methylgruppe bedeuten.

9. C-Glycosid-Derivat der Formel (III): 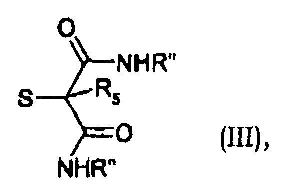 worin bedeuten:
- S ein Monosaccharid oder ein Polysaccharid mit bis zu 20 Zuckereinheiten in Pyranose- und/oder Furanose-Form sowie in L- und/oder D-Form, wobei das Monosaccharid oder Polysaccharid mindestens eine freie Hydroxylfunktion aufweist,
- die Bindung S-C- eine C-Anomer-Bindung,
- R₅ :
eine gesättigte oder ungesättigte, geradkettige, cyclische oder verzweigte, unsubstituierte C₁-C₁₂-Alkylgruppe oder
eine Benzylgruppe oder
ein Halogenatom;
- R" ein Wasserstoffatom oder eine gesättigte oder ungesättigte, geradkettige, cyclische oder verzweigte, unsubstituierte C₁-C₁₂-alkylgruppe.

10. C-Glycosid-Derivat nach Anspruch 9, **dadurch gekennzeichnet, dass** es unter folgenden Verbindungen ausgewählt ist:

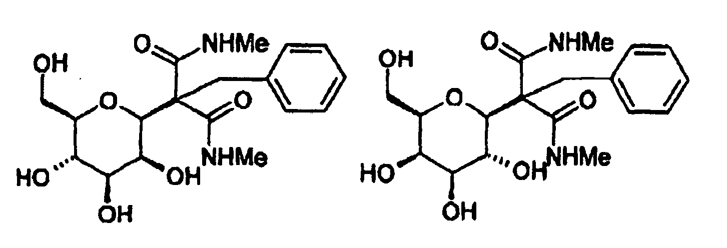

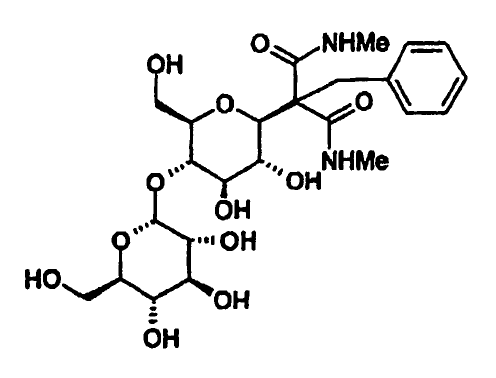

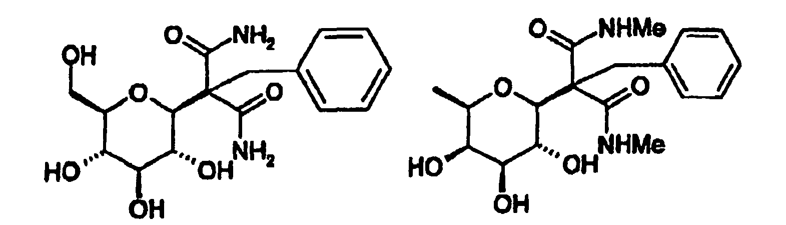

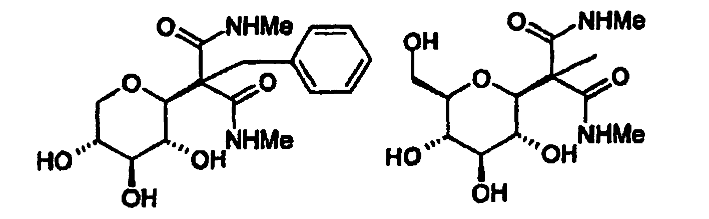

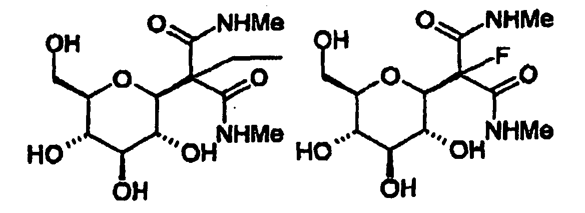

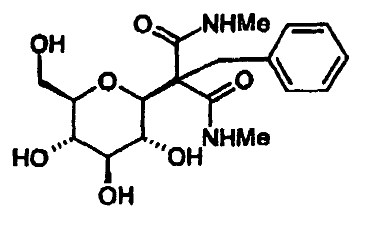

11. C-Glycosid-Derivat nach Anspruch 9 oder 10, **dadurch gekennzeichnet, dass** R₅ eine Benzylgruppe oder eine Methylgruppe und R" eine Methylgruppe bedeuten.

12. Verfahren zur kosmetischen nichttherapeutischen Behandlung der Haut oder der Kopfhaut, das die topische Anwendung einer Zusammensetzung wie in einem der Ansprüche 1 bis 8 definiert auf die Haut oder die Kopfhaut umfasst.

13. Kosmetisches nichttherapeutisches Verfahren zur Vorbeugung oder Milderung der Zeichen der Hautalterung und/oder für einen strahlenderen Teint und/oder zur Bekämpfung trockener Haut, das die topische Anwendung einer Zusammensetzung wie in einem der Ansprüche 1 bis 8 definiert auf die Haut umfasst.

14. Verwendung der in einem der Ansprüche 1 bis 8 definierten Zusammensetzung zur Herstellung einer dermatologischen Präparation zur Behandlung von Akne oder Hyperkeratosen.

15. Kosmetisches nichttherapeutisches Verfahren zum Schutz der Haut gegen schädliche Einflüsse von UV-Strahlung und Umweltverunreinigungen, das die topische Anwendung einer Zusammensetzung nach einem der Ansprüche 1 bis 8 auf die Haut umfasst.

16. Kosmetisches nichttherapeutisches Verfahren zur Verbesserung der Barrierefunktion der Haut und/oder der Hydratation der Haut, das die topische Anwendung einer Zusammensetzung nach einem der Ansprüche 1 bis 8 auf die Haut umfasst.
